# EUROPÄISCHE PATENTANMELDUNG

(11) **EP 0 557 712 A1**
(43) Veröffentlichungstag der Anmeldung: **01.09.1993**
(21) Anmeldenummer: 93100774.4
(22) Anmeldetag: 20.01.1993
(51) Int. Cl.: A61F 5/01

(54) **Spreizschiene**

(30) Priorität: 25.02.1992 DE 4205716
(71) Anmelder: TRIMBORN MASCHINEN- UND APPARATEBAU GmbH, D-53879 Euskirchen (DE)
(72) Erfinder: Kuerdt, Stefan, Dr. med., W-5000 Köln 41 (DE); Ellerich, Karl J., Dr. med., W-5042 Erftstadt (DE)
(74) Vertreter: Mayer-Pohske, Joachim-Wolfgang

(57) **Zusammenfassung**

Die Erfindung betrifft eine Spreizschiene (1') zur Korrektur von Fehlstellungen des Hüftgelenkes bei Kleinkindern, mit einer Beckenplatte (2) mit einer Vorder- und einer Rückseite und einem daran angebrachten Beckengurthalter (3) sowie mit zwei auf der Vorderseite angeordneten Oberschenkelschalen (9',10'). Um eine Spreizschiene preiswert herstellen zu können, die dazu noch einfach in der Handhabung ist und im angelegten Zustand die Überprüfung der Stellung des Hüftgelenkes durch Röntgenaufnahmen ohne störende Reflexionen erlaubt, wird vorgeschlagen, daß die gesamte Spreizschiene aus einem von Röntgenstrahlen durchstrahlbaren Material hergestellt und die Beckenplatte mit dem Beckengurthalter einstückig ausgebildet und beidseitig einer Längsachse um einen Einstellbereich eines Beugungswinkels vergrößert ist, wobei die Beckenplatte beidseitig ihrer Längsachse in spiegelbildlicher Anordnung je ausgehend von einem Zentrum und radial verlaufend eine Vielzahl von Bohrungen aufweist, und daß mindestens die Oberschenkelschalen je mit einem einen Neigungswinkel aufweisenden Halter verbunden sind und die Halter je an einer ebenen Unterseite an die Lage und den Durchmesser der Bohrungen angepaßte Sockelbohrungen aufweisen, von denen mindestens eine mit einem Gewinde (17,20) versehen ist, wobei die Halter auf der Vorderseite der Beckenplatte vorzugsweise über Stifte und Befestigungsschrauben befestigbar sind.

## Beschreibung

Die Erfindung betrifft eine Spreizschiene zur Korrektur von Fehlstellungen des Hüftgelenkes bei Kleinkindern, mit einer Beckenplatte mit einer Vorder- und einer Rückseite und einem daran angebrachten Beckengurthalter sowie mit zwei auf der Vorderseite getragenen Oberschenkelschalen.

Spreizschienen der o.g. Art bestehen aus sehr vielen Bauteilen, die über Verschraubungen miteinander verbunden sind. Besonders die Konstruktion der die Oberschenkelschalen tragenden Einstellvorrichtungen ist sehr aufwendig. Bedingt durch die aufwendige Konstruktion ist die Herstellung derartiger Spreizschienen teuer. Auch die Handhabung der Spreizschienen bzw. die korrekte Einstellung der gewünschten Spreizbreite und des Beugungswinkels der Oberschenkel des Kleinkindes, zur Korrektur der Fehlstellung des Hüftgelenkes, ist durch die Vielzahl der zu lösenden und festzustellenden Verschraubungen nicht einfach. Ein weiterer Nachteil der bekannten Einrichtung liegt auch darin, daß die Bauteile der Einrichtung so angeordnet und gestaltet sind, daß sie bei Röntgenaufnahmen an den zu prüfenden Stellen störende Reflexionen auslösen.

Der Erfindung liegt, ausgehend von dieser Situation, die Aufgabe zugrunde, eine Spreizschiene vorzuschlagen, die aus wenigen Bauteilen preiswert hergestellt werden kann, einfach in der Handhabung ist und im angelegten Zustand die Überprüfung der Stellung des Hüftgelenkes durch Röntgenaufnahmen ohne störende Reflexionen in den zu überprüfenden Bereichen erlaubt.

Erfindungsgemäß ist diese Aufgabe dadurch gelöst, daß die gesamte Spreizschiene aus einem von Röntgenstrahlen durchstrahlbaren Material hergestellt, und die Beckenplatte mit dem Beckengurthalter einstückig ausgebildet und beidseitig einer Längsachse um einen Einstellbereich eines Beugungswinkels vergrößert ist, wobei die Beckenplatte beidseitig ihrer Längsachse in spiegelbildlicher Anordnung je ausgehend von einem Zentrum und radial verlaufend eine Vielzahl von Bohrungen aufweist, und daß mindestens die Oberschenkelschalen je mit einem einen Neigungswinkel aufweisenden Halter verbunden sind und die Halter je an einer ebenen Unterseite an die Lage und den Durchmesser der Bohrungen angepaßte Sockelbohrungen aufweisen, von denen mindestens eine mit einem Gewinde versehen ist, wobei die Halter auf der Vorderseite der Beckenplatte mindestens über eine Befestigungsschraube befestigbar sind. Hierdurch wird die Zahl der Bauteile der Spreizschiene reduziert und gleichzeitig die Herstellungskosten gesenkt. Trotz des einfachen Aufbaus sind mit der erfindungsgemäßen Spreizschiene alle für die Korrektur des Hüftgelenkes wesentlichen Einstellmöglichkeiten durchführbar, wodurch die Handhabung bzw. die individuelle Anpassung der Spreizschiene sehr vereinfacht wird. Der einfache Aufbau ermöglicht es weiterhin, sämtliche Bauteile der Spreizschiene aus einem für Röntgenstrahlen durchstrahlbaren Material herzustellen. Besonders günstig ist es, wenn der Halter außer dem Gewinde für eine Befestigungsschraube noch einen Stift für eine Arretierung aufweist, so daß dieser Stift in eine zugeordnete Bohrung auf der Vorderseite der Beckenplatte eingesetzt werden kann. Diese Einrichtung ist außerdem, wie unten noch dargelegt wird, ergänzbar, so daß auch schwerste Hüftgelenkfehler behandelbar werden.

Nach einer Ausgestaltung der Erfindung ist vorgeschlagen, daß von den Zentren je in entgegengesetzten Richtungen fächerförmig gedachte Linien ausgehen, auf denen sich in radialer Verteilung die Bohrungen befinden, wobei die gesamten Linien oberhalb einer die Zentren verbindenden Achse einen Winkel von vorzugsweise 30° und unterhalb dieser Achse einen Winkel von vorzugsweise 60° einnehmen. Durch die einfache Befestigung der aus Halter und Oberschenkelschale gebildeten baulichen Einheit mit Hilfe dieser Bohrungen kann sehr schnell die korrekte Einstellung des Beugungswinkels der Oberschenkelschalen bzw. der von den Oberschenkelschalen gehaltenen Extremitäten vorgenommen werden.

Nach einer weiteren Ausgestaltung der Erfindung ist vorgeschlagen, daß die Zahl der Bohrungen entlang jeder gedachten Linie einer geraden Zahl entspricht und die Bohrungen in zwei gleich große Gruppen geteilt sind, wovon die Bohrungen mindestens einer Gruppe durchgehend sind und auf der Rückseite der Beckenplatte je eine Senkung aufweisen, wobei die Sockelbohrungen der Halter einen Abstand aufweisen, der mit den Bohrungen der Beckenplatte korrespondiert. Durch verschieben der Halter entlang je einer der gedachten Linien in radialer Richtung kann die Spreizbreite bzw. der Neigungswinkel der von den Oberschenkelschalen gehaltenen Extremitäten stufenweise verändert werden, wobei der Halter zunächst mit einem in die Sockelbohrung und der damit korrespondierenden Bohrung der Beckenplatte eingeführten Stift in der gewünschten Position fixiert und dann mit einer durch die durchgehende Bohrung der Beckenplatte geführten Schraube aus durchstrahlbarem Material auf der Vorderseite der Beckenplatte befestigt wird.

Ferner ist nach einer Ausgestaltung der Erfindung vorgeschlagen, daß die Spreizschiene auf mindestens der Vorderseite der Beckenplatte mindestens teilweise mit einer hautfreundlichen, für Röntgenstrahlen durchlässigen Polsterung versehen ist. Hierdurch wird das Tragen der Spreizschiene für die Kleinkinder angenehmer und mögliche Druckstellen oder Scheuerstellen weitgehend vermieden.

Nach einer ergänzenden Ausgestaltung der Erfindung ist vorgeschlagen ,daß am Halter eine Fläche vorgesehen ist, die mit der Horizontalen einen Neigungswinkel bildet, wobei diese Fläche entlang ihrer steilsten Neigung mindestens eine erste Führungsnut aufweist, in welche ein der Oberschenkelschale zugeordnetes, entsprechend geformtes Gegenstück eingesetzt ist, wobei die Oberschenkelschale mit einer ebenen Gegenfläche an der Fläche anliegt und auf ihr verschiebbar und in gewünschter Position feststellbar ist. Hierdurch werden die Einstellmöglichkeit und damit der Einsatzbereich bei Hüftgelenksfehlstellungen unterschiedlichster Art deutlich erweitert.

In weiterer Ergänzung der Erfindung ist vorgeschlagen, daß parallel und im Abstand zur ersten Führungsnut eine weitere Führungsnut vorgesehen ist, in welche ein einer Unterschenkelschale zugeordnetes, entsprechend geformtes Gegenstück eingesetzt ist, wobei die Unterschenkelschale in Richtung der Fläche verschiebbar und in gewünschter Position feststellbar ist. Die zusätzliche Verwendung einer verstellbaren und in gewünschter Position festsetzbaren Unterschenkelschale in Verbindung mit einer ebenfalls entsprechend verstellbaren und feststellbaren Oberschenkelschale macht die Behandlung selbst schwerster Hüftgelenksfehler möglich, die bisher nur durch eine Gipsfestlegung der Gelenke behandelt werden konnten. Solche Gipsfestlegungen aber sind außerordentlich nachteilig, weil sie nach Anlage und Aushärtung einerseits nicht ständig abgenommen werden können zur Reinigung und weil andererseits nicht sichergestellt ist, daß die richtigen Gelenkpositionen nach Aushärtung des Gipses auch tatsächlich erreicht worden sind. Irgendwelche Korrekturmöglichkeiten existieren nicht.

Ausgestaltend ist dann nach der Erfindung noch vorgeschlagen, daß das Gegenstück mit mindestens einer Schraube an der zugeordneten Schale befestigt ist. Hierdurch kann die zugeordnete Schale jeweils zusätzlich um die Schraubenachse als Drehachse gedreht bzw. geschwenkt werden, wodurch eine zusätzliche Anpassungsmöglichkeit entsteht.

Eine andere Alternative der Erfindung schlägt vor, daß das Gegenstück und die zugeordnete Schale einstückig ausgebildet sind. Eine solche einstückige Ausbildung ist vorteilhaft dann, wenn bekannte und häufig auftretende Fehlstellungen korrigiert werden sollen. Einer zusätzlichen Einstellmöglichkeit bedarf es nicht, so daß die Verwendung einer Schale mit einstückig daran ausgebildetem Gegenstück die Anwendung der gesamten Spreizschiene und deren Einstellung erleichtert.

Eine Variante der Erfindung sieht vor, daß mindestens die Oberschenkelschalen und die Halter einstückig ausgebildet sind. Hierdurch wird die richtige Einstellung der Oberschenkelschalen sehr erleichtert und es wird nicht das Risiko eingegangen, daß im Falle einer zweistückigen Ausbildung sich die entsprechende Verbindungsschraube löst und hierdurch eine Fehleinstellung der Oberschenkelschale hervorruft. Dadurch, daß die Oberschenkelschalen mit dem zugeordneten Halter einstückig ausgebildet sind, wird die Anordnung sehr formstabil und es wird jedes Risiko einer Fehleinstellung vermieden. Um unterschiedliche Forderungen erfüllen zu können, können hierbei die Oberschenkelschalen und die Halter in unterschiedlichen Größen und Lagen hergestellt werden, so daß sie in dieser einstückigen Ausbildung nach ihrer Lage und Neigung der Oberschenkelschalen unterschieden und ausgesucht und eingesetzt werden können. Bei Bedarf können zusätzlich Unterschenkelschalen vorgesehen sein, die entweder verschiebbar und einstellbar oder ebenfalls in einer bevorzugten Lage und Neigung einstückig mit dem genannten Halter ausgebildet sein können. Da diese Teile in unterschiedlichen Neigungen und unterschiedlichen Größen hergestellt und auf Vorrat gehalten werden können, kann ein einfacher Austausch dieser Teile eine Anpassung an z. B. das Wachstum eines behandelten Kindes oder eine Anpassung an eine verbesserte Situation des Hüftgelenkschadens ermöglichen.

Schließlich ist noch vorgeschlagen, daß die Halter in den Führungsnuten geschlitzt sind, wobei die Schlitze und damit die Führungsnuten über quer eingesetzte Schrauben zusammenziehbar sind zur Festklemmung der jeweils geführten Schale. Dies ist eine sehr einfache Möglichkeit, die genannten Schalen in jeder beliebigen Position entlang der Führungsnuten sicher festzuklemmen.

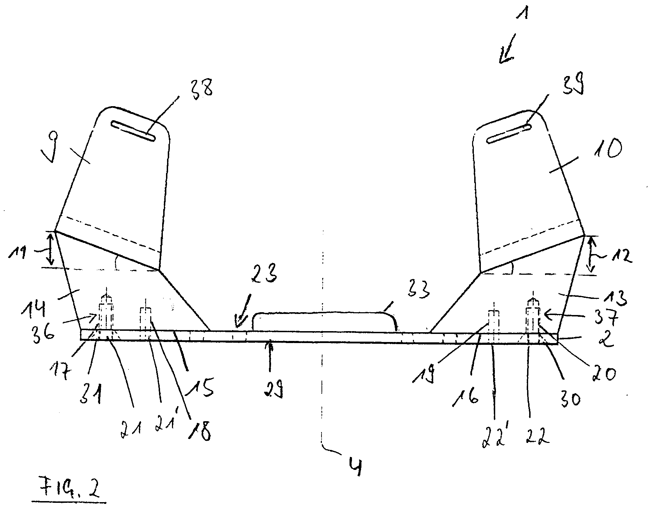

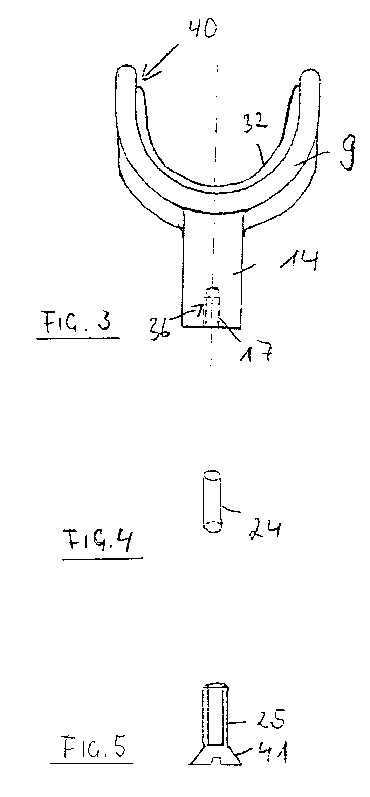

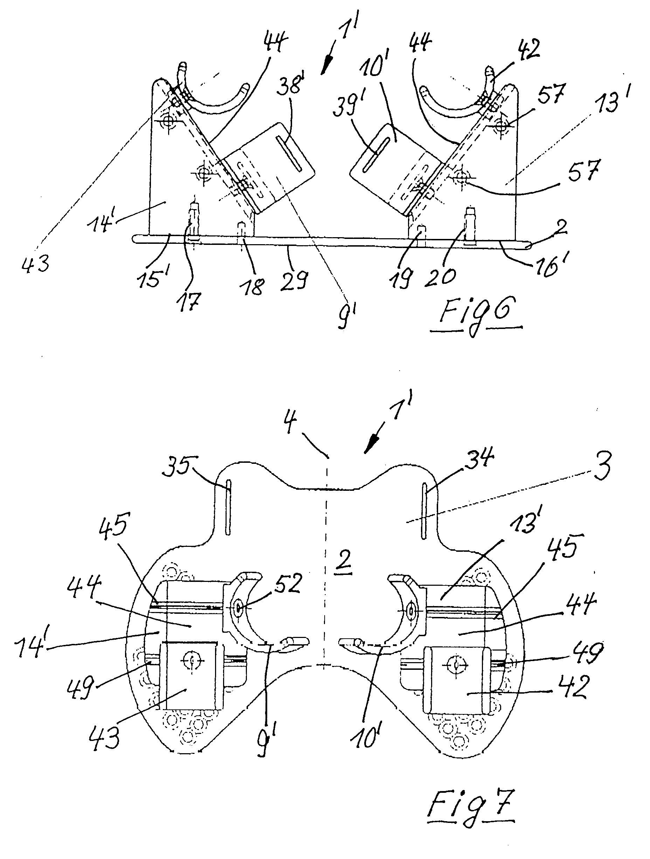

Die Erfindung soll nun anhand der beigefügten Zeichnungen näher erläutert werden. Es zeigen:
- Figur 1: die einstückige Ausbildung von Beckenplatte und Beckengurthalter in der Draufsicht
- Figur 2: eine Spreizschiene in der Vorderansicht
- Figur 3: die bauliche Einheit aus Oberschenkelschale und Fußsockel in der Vorderansicht
- Figur 4: einen Stift in der Seitenansicht
- Figur 5: eine Schraube in der Seitenansicht
- Figur 6: Variante einer Spreizschiene in Ansicht wie Fig. 2
- Figur 7: Draufsicht nach Fig. 6
- Figur 8: Variante eines Halters in Ansicht des Pfeils A nach Fig. 9
- Figur 9: Ansicht in Richtung des Pfeils B nach Fig. 8
- Figur 10: Ansicht wie Fig. 9 mit in die Unterseite eingebrachte Befestigungsbohrungen
- Figur 11: Ansicht in Richtung des Pfeils C nach Fig. 9
- Figur 12: Unterschenkelschale in Ansicht des Pfeils E nach Fig. 13
- Figur 13: Unterschenkelschale in Ansicht des Pfeils D nach Fig. 12
- Figur 14: Variante Unterschenkelschale in Ansicht des Pfeils G nach Fig. 15
- Figur 15: Variante Unterschenkelschale in Ansicht des Pfeils F nach Fig. 14
- Figur 16: Gegenstück für Führungsnut in Ansicht des Pfeils H nach Fig. 14
- Figur 17: Variante Oberschenkelschale einstückig mit Gegenstück in Ansicht des Pfeils L nach Fig. 18
- Figur 18: Oberschenkelschale in Ansicht des Pfeils K nach Fig. 17
- Figur 19: Ansicht wie Fig. 17, jedoch mit angeschraubtem Gegenstück
- Figur 20: Ansicht in Richtung des Pfeils M nach Fig. 19

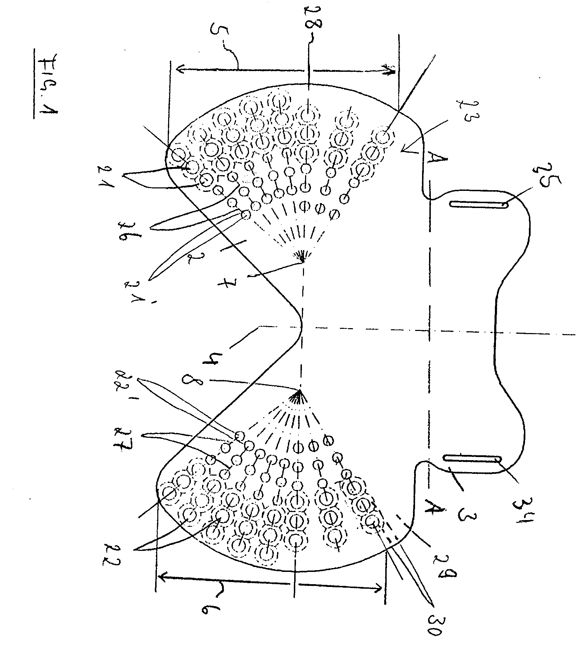

Figur 1 zeigt die Vorderseite 23 der Beckenplatte 2 und des Beckengurthalters 3, die einstückig ausgebildet sind. Die Abgrenzung zueinander ist durch die gestrichelte Linie A-A angedeutet. Der Beckengurthalter 3 zeigt Führungsschlitze 34,35 für einen in Figur 1 nicht dargestellten Beckengurt. Beidseitig der Längsachse 4 befinden sich in spiegelbildlicher Anordnung, auf von den Zentren 7,8 ausgehenden gedachten Linien 26,27, in radialer Verteilung Bohrungen 21,22;21'22'. Die Anzahlder Bohrungen 21,22;21',22' auf einer gedachten Linie 26,27 entspricht einer geraden Zahl. Die Bohrungen 21,22;21',22' sind in zwei gleich große Gruppen geteilt, wovon eine Gruppe durchgehende Bohrungen 21,22 aufweist, die auf der Rückseite 29 der Beckenplatte 2 mit Senkungen 30 versehen sind. Beidseitig der Längsachse 4 nehmen die gedachten Linien 26,27 oberhalb der die Zentren 7,8 verbindenden Achse 28 einen Winkel von vorzugsweise 30° und unterhalb der Achse 28 einen Winkel von vorzugsweise 60° ein. Beide Winkel zusammen ergeben beidseitig der Längsachse 4 den Einstellbereich 5,6 des Beugungswinkels.
Die einstückige Ausbildung aus Beckenplatte 2 und Beckengurthalter 3 ist aus einem für Röntgenstrahlen durchlässigen Material gefertigt. Die Beckenplatte 2 und/oder der Beckengurthalter 3 kann in verschiedenen Baugrößen hergestellt werden, womit eine optimale Anpassung an die jeweilige Körpergröße eines Kindes möglich ist.
Durch die Befestigung der in Figur 2 und 3 gezeigten, aus Halter 13,14 und Oberschenkelschale 9,10 gebildeten baulichen Einheit entlang der vorgegebenen, gedachten Linien 26,27 kann sehr schnell die korrekte Einstellung des Beugungswinkels 5,6 der Oberschenkelschalen 9,10, bzw. der von den Oberschenkelschalen 9,10 gehaltenen Extremitäten vorgenommen werden. Durch Verschieben der Halter 13,14 entlang je einer der gedachten Linien 26,27 in radialer Richtung kann die Spreizbreite bzw. der Neigungswinkel der von den Oberschenkelschalen 9,10 gehaltenen Extremitäten verändert werden, wobei die Halter 13,14 zunächst mit in Figur 4 gezeigten, in die in Figur 2 gezeigten Sockelbohrungen 18,19 und den damit korrespondierenden Bohrungen 21',22' der Beckenplatte 2 eingeführten Stiften 24 in der gewünschten Position fixiert und dann mit durch die durchgehenden Bohrungen 21,22 der Beckenplatte 2 geführten, in Figur 5 gezeigten Schrauben 25 auf der Vorderseite 23 der Beckenplatte 2 befestigt werden.

Figur 2 zeigt eine Spreizschiene 1 in der Seitenansicht. Die beidseitig der Längsachse 4 auf der Vorderseite 23 der Beckenplatte 2 befestigten Oberschenkelschalen 9,10 bilden mit den einen Neigungswinkel 11,12 aufweisenden Haltern 13,14 eine bauliche Einheit. Die Oberschenkelschalen 9,10 zeigen Halteschlitze 38,39 für in der Figur 1 nicht dargestellte Oberschenkelgurte. Die Halter 13,14 weisen an ihren ebenen Unterseiten 15,16 in der Lage und im Durchmesser an die Bohrungen 21,22, 21'22' angepaßte Sockelbohrungen 17 bis 20 auf, von denen je Halter 13,14 mindestens eine mit einem Gewinde 36,37 versehen ist. Mindestens die auf der Rückseite 29 der Beckenplatte 2 mit einer Senkung 30,31 versehenen Bohrungen 21,22 sind durchgehend. Die Halter 13,14 sind über die in Figur 4 gezeigten Stifte 24 und über die in Figur 5 gezeigten Schrauben 25 mit ihrer Unterseite 15,16, an den Bohrungen 21,22,21',22' und Sockelbohrungen 17 bis 20 auf der Vorderseite 23 der Beckenplatte 2 befestigt. Aus Gründen der Übersichtlichkeit sind in der Figur 2 beidseitig der Längsmittelachse 4 jeweils nur zwei Bohrungen 21,22,21',22' in der Beckenplatte 2 dargestellt. Die Lage der weiteren Bohrungen auf der in Figur 1 gezeigten, gedachten Linie 26,27 ist durch gestrichelte Linien angedeutet. Zusätzlich zeigt die Vorderseite 23 der Beckenplatte 2 eine Polsterung 33.
Die Zahl der Bauteile der Spreizschiene 1 ist somit stark reduziert. Trotz des einfachen Aufbaus der Spreizschiene 1 sind alle für die Korrektur des Hüftgelenkes wesentlichen Einstellmöglichkeiten durchführbar, wodurch die Handhabung bzw. die individuelle Anpassung der Spreizschiene 1 sehr vereinfacht wird. Der einfache Aufbau ermöglicht es weiterhin, sämtliche Bauteile der Spreizschiene 2 aus einem für Röntgenstrahlen durchstrahlbaren Material herzustellen. Dadurch daß die Oberflächen der Spreizschiene 1, mit denen die Kleinkinder beim Tragen der Spreizschiene 1 in Berührung kommen, mit einer hautfreundlichen Polsterung 33 versehen sind, ist für die Kleinkinder das Tragen der Spreizschiene 1 angenehm und mögliche Druckstellen oder Scheuerstellen werden weitgehend vermieden.

Figur 3 zeigt die aus der Oberschenkelschale 9 und dem Halter 14 gebildete bauliche Einheit in der Vorderansicht. Die Innenseite 40 der Oberschenkelschale 9 ist mit einer Polsterung 32 versehen. Der Halter 14 zeigt noch einmal aus einer anderen Sicht die Lage der Sockelbohrung 17 mit dem Gewinde 36. Auch die Oberschenkelschalen 9 bzw. 10 können in verschiedenen Baugrößen hergestellt und damit der Körpergröße der Kleinkinder angepaßt werden. Dabei ist es auch möglich, die Halter 14 mit unterschiedlichen, in Figur 2 gezeigten Neigungswinkeln 11,12 zu versehen. Durch die Kombination der in verschiedenen Baugrößen hergestellten und mit unterschiedlichen Neigungswinkeln versehenen Oberschenkelschalen 9,10 bzw. Halter 13,14 mit den ebenfalls in verschiedenen Baugrößen hergestellten Beckenplatten 2 bzw. den Beckengurthaltern 3 ist eine erstaunlich große Zahl von Anpassungsmöglichkeiten an die jeweiligen individuellen Voraussetzungen gegeben. Durch die hautfreundliche Polsterung 32 an den Oberflächen der Oberschenkelschalen 9,10 die mit dem Kleinkind in Berührung kommen, wird auch hier der Tragekomfort verbessert.

Figur 4 und 5 zeigen einen Stift 24 und eine Schraube 25 in der Seitenansicht. Auch die Stifte 24 und die Schrauben 25 sind aus einem für Röntgenstrahlen durchlässigen Material hergestellt. Die Stifte 24 sind in ihrer Länge der Tiefe der in Figur 1 und 2 gezeigten Bohrungen 21',22' und der Tiefe der Sockelbohrungen 18,19 angepaßt. Dadurch, daß die Bohrungen 21',22' zur Unterseite 29 der Beckenplatte 2 nicht durchgehend sind, sind die eingesetzten Stifte 24 gegen ein Herausfallen gesichert. Die Schrauben 25 sind mit einem an die Senkungen 30,31 angepaßten Senkkopf 41 versehen, wodurch ein Überstand der eingeschraubten Schrauben 25 an der Rückseite 29 der Beckenplatte 2 vermieden wird.

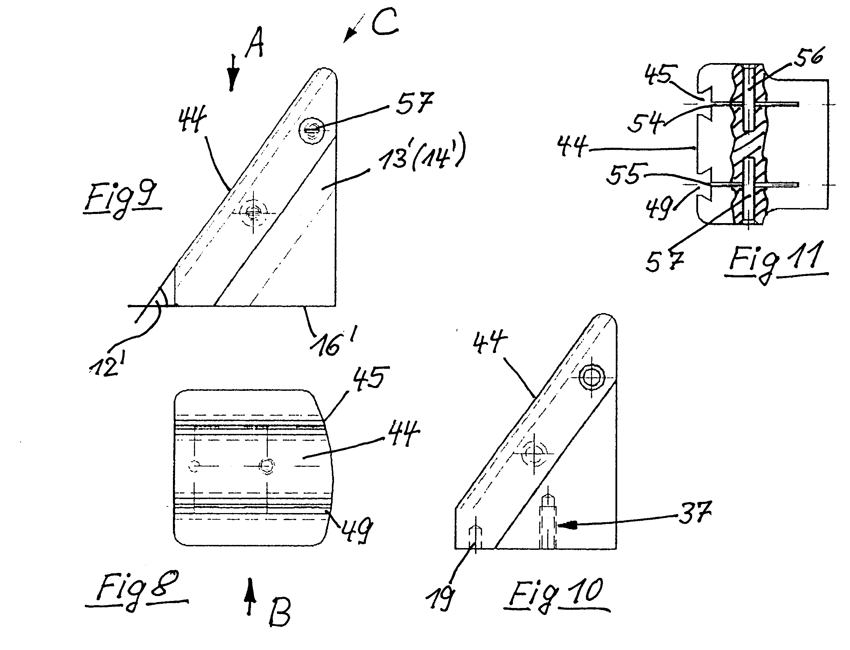

Die Figuren 6 bis 20 zeigen einen Aufbau der bereits beschriebenen Spreizschiene, bei der die Oberschenkelschalen nicht mehr einstückig mit dem zugeordneten Halter verbunden sind und bei der weiter gesonderte Unterschenkelschalen vorgesehen sind. Hierzu wird wieder die bereits beschriebene Beckenplatte 2 in der bereits beschriebenen Ausführung verwendet. Anstelle der bereits beschriebenen Halter 13 und 14 sind nunmehr Halter 13' und 14' vorgesehen, die allerdings ebenso an der Beckenplatte 2 verstellt und befestigt werden, wie dies bereits zu den Haltern 13 und 14 beschrieben wurde. Die in der Bauvariante nach den Figuren 6 bis 20 verwendeten Halter 13' bzw. 14' sind gesondert dargestellt in den Figuren 8 bis 11. Hier ist zu erkennen, daß die Halter 13' und 14' identisch ausgebildet sein können, so daß in den Figuren 8 bis 11 nur ein solcher Halter 13' dargestellt ist. Dieser Halter 13' ist ebenfalls aus einem geeigneten Kunststoff geformt und weist eine der Unterseite 16 des Halters 13 entsprechende Unterseite 16' auf. Sieht man diese Unterseite 16' als eine horizontale Ebene an, so weist der Halter 13' eine Fläche 44 auf, wobei diese Fläche 44 unter einem Neigungswinkel 12' zur genannten Horizontalen verläuft. Dieser Neigungswinkel 12' kann bei Bedarf sehr groß und damit die Fläche 44 sehr steil liegend vorgesehen sein. Diese Fläche 44 weist parallel zu einer Linie unter dem Neigungswinkel 12' zwei parallel zueinander und im Abstand zueinander verlaufende Führungsnuten 45 und 49 auf, die als Schwalbenschwanznuten ausgebildet sind. Am Grund dieser Nuten sind tief in den Halter 13' eindringende Schlitze 54 und 55 vorgesehen, die über quer eingesetzte Schrauben 56 und 57 zusammenziehbar sind. Hierdurch werden die Führungsnuten 45 und 49 in ihrer Breite verkleinert, so daß sich ein darin angeordnetes Gegenstück festklemmen läßt.

Die Figuren 6 und 7 zeigen in Vorderansicht und Draufsicht eine zusammengebaute Spreizschiene 1', die anstelle der oben bereits beschriebenen Halter 13 und 14 die zu den Figuren 8 bis 11 beschriebenen Halter 13' und 14', die untereinander identisch sein können, aufweist, wobei diese Halter 13' und 14' auf der Beckenplatte 2 ebenso befestigt sind, wie die Halter 13 und 14, so daß hier diese Befestigung nicht mehr erneut beschrieben werden muß.

In die Führungsnuten 45 und 49 sind in dem in der Figur 6 und 7 dargestellten Ausführungsbeispiel die entsprechend schwalbenschwanzförmigen Gegenstücke 47 bzw. 51 eingesetzt, die mit einer Oberschenkelschale 9' bzw. mit einer Unterschenkelschale 42 über eine Schraube 52 bzw. 53 lösbar verbunden sind. Hierbei muß beachtet werden, daß sich wegen der identischen Ausbildung der Halter 13' bzw. 14' die Bezeichnung der Führungsnuten 45 bzw. 49 umkehren kann, je nach dem, ob der Halter in Figur 7 als rechter Halter 13' oder als linker Halter 14' eingesetzt ist.

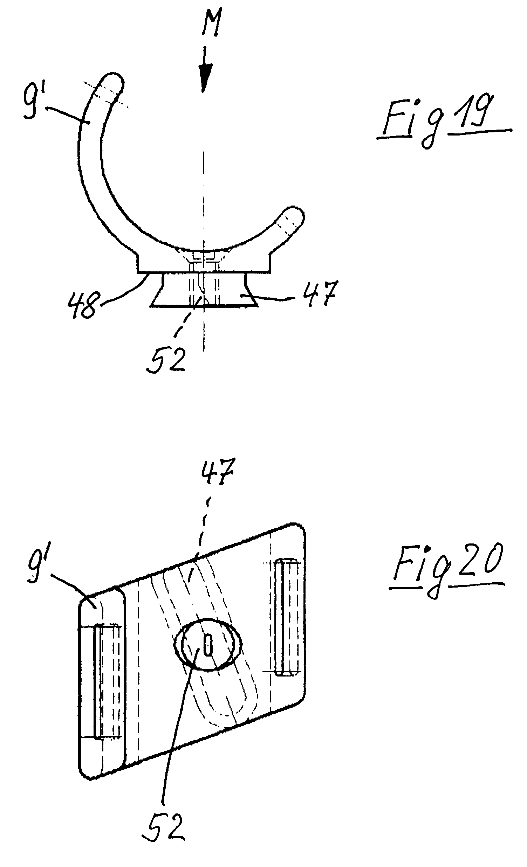

Die Figuren 19 und 20 zeigen jeweils in Vorderansicht und in Draufsicht eine Oberschenkelschale 9', über die Schraube 52 lösbar mit dem Gegenstück 47 verbunden und auf der linken Seite nach Figur 7 am Halter 14' eingesetzt und angeordnet. Das Gegenstück hierzu ist die Oberschenkelschale 10', die in Figur 7 am rechten Halter 13' entsprechend angeordnet ist. Die Oberschenkelschale 9' und 10' ist, wie dies in Figur 20 zu erkennen ist, gegenüber dem Gegenstück 47 etwas verdreht angeordnet und es ist der Schalenteil in der Art der Steigung eines Gewindes schraubenartig verformt gestaltet. Diese gewindeartige Steigung ist nicht zwingend. Die Schale kann auch so gestaltet sein, daß diese erwähnte Gewindesteigung gleich Null ist. Die Ausformung jedoch, wie in Figur 20 zu erkennen, ergibt im Einsatz am Körper des Kleinkindes Platzvorteile, so daß auch hierdurch in bestimmten Fällen Druckstellen vermieden werden können. Die in entsprechender Anordnung in den Figuren 6 und 7 gezeigten Oberschenkelschalen 9' und 10' weisen die genannte und in Figur 20 zu erkennende Steigung nicht auf.

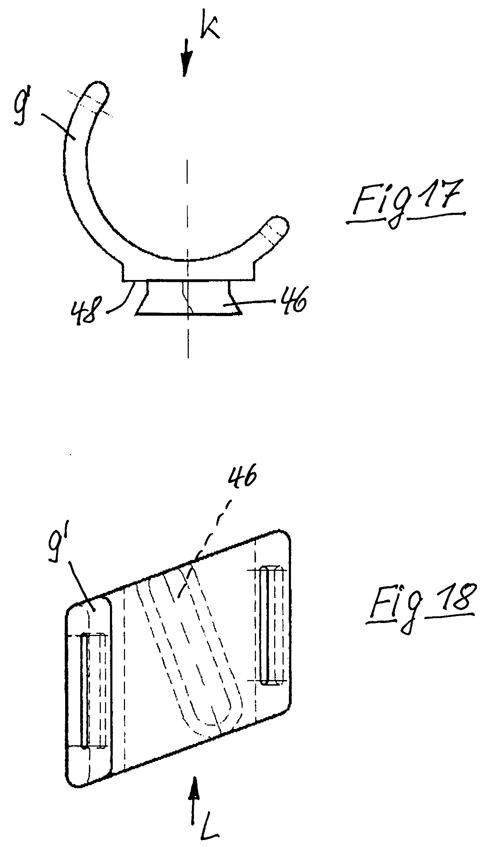

In den Figuren 17 und 18 ist eine Oberschenkelschale dargestellt, die der Oberschenkelschale nach den Figuren 19 und 20 entspricht. Jedoch ist die Oberschenkelschale nach den Figuren 17 und 18 mit dem Gegenstück 46 einstückig verbunden.

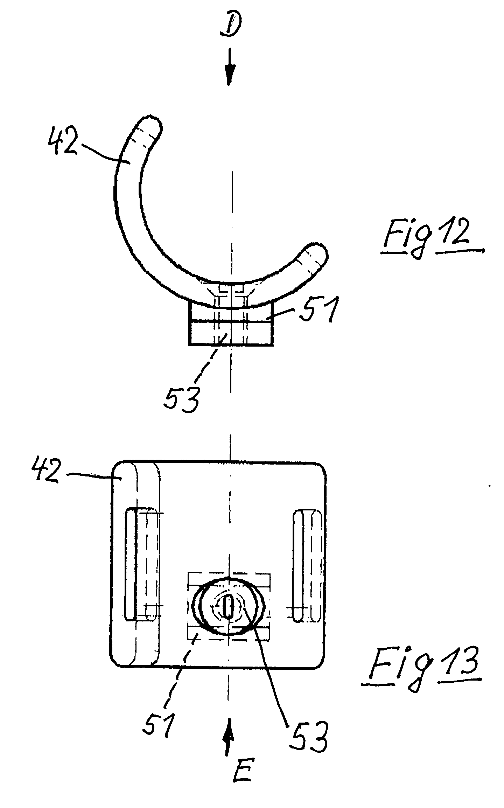

Die Oberschenkelschale 10' auf der rechten Seite in Figur 7 kann entlang der Führungsnut 45 verschoben werden. Hierbei kann diese Oberschenkelschale 10' auf der Fläche 44 mit ihrer auf der Unterseite vorgesehenen Gegenfläche 48 gleiten. In einer gewünschten Stellung der Oberschenkelschale 10' (oder natürlich auch 9') kann dann die Schraube 56 (siehe Figur 11) angezogen werden, so daß die Oberschenkelschale 10' hierdurch in der gewünschten Position sicher geklemmt ist.

In der weiteren an den Haltern 13' bzw. 14' vorgesehenen Führungsnut 49 ist im Ausführungsbeispiel nach den Figuren 6 und 7 jeweils das Gegenstück 51 eingeschoben, daß über eine Schraube 53 mit einer Unterschenkelschale 42 oder 43 verbunden ist. Diese Verbindung ist in den Figuren 12 und 13 dargestellt. Dort ist zu erkennen, daß die Unterschenkelschale 42 mit Hilfe der Schraube 53 am Gegenstück 51 befestigt ist und hierbei in irgendeiner gewünschten Drehlage festgestellt werden kann. Auch die Unterschenkelschalen 42 bzw. 43 können in der zugeordneten Führungsnut 49 verschoben und an beliebiger Stelle durch Anziehen der Schraube 57 (siehe Figur 11) festgeklemmt werden.

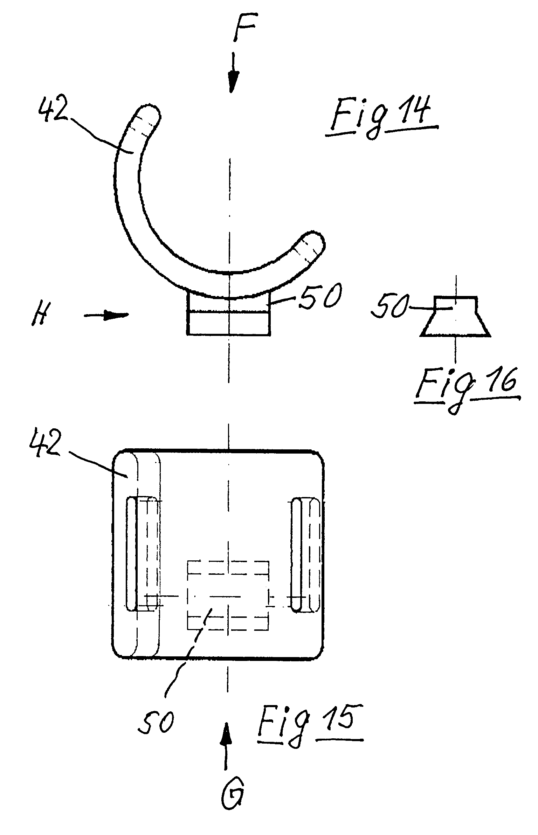

Die Unterschenkelschalen 42 bzw. 43 können natürlich ebenfalls mit dem zugeordneten Gegenstück 50 einstückig ausgebildet sein, wie dies in den Figuren 14 und 15 dargestellt ist, wobei Figur 16 noch einmal den schwalbenschwanzförmigen Querschnitt des Gegenstückes 15 zeigt. Bei einstückiger Ausführung ist natürlich eine Veränderung der Drehwinkellage der Unterschenkelschale 42 nicht mehr möglich. Die Ausführungsform nach Figur 6 und 7 muß nicht zwingend Unterschenkelschalen aufweisen, sondern kann sich auch auf den Einsatz von Oberschenkelschalen beschränken und in dieser Beschränkung der Ausführungsform nach Figur 2 entsprechen, hierbei aber größere Anpassungsmöglichkeiten aufweisen. Die Ergänzung mit entsprechend einstellbaren Unterschenkelschalen sorgt zusammen mit den Einstellmöglichkeiten der Oberschenkelschalen in der Ausführungsform nach den Figuren 6 und 7 dafür, daß selbst extreme Hüftgelenksfehlbildungen behandelt werden können, so daß die in solchen Fällen bisher notwendige und sehr unhygienische Eingipsung vermieden werden kann. Außerdem ist bei der Ausführungsform nach den Figuren 6 und 7 nach der Einstellung die erreichte Lage überprüfbar und ggfls. korrigierbar. Bei der Anwendung von Gips ist eine solche Korrektur nicht möglich.

Mit der Erfindung ist somit eine Spreizschiene bekannt geworden, die aus wenigen Bauteilen preiswert hergestellt werden kann und außerdem einfach in der Handhabung ist. Dadurch, daß die Bauteile der erfindungsgemäßen Spreizschiene in unterschiedlichen Größen hergestellt und kombiniert werden können, ist eine erstaunlich große Zahl von Anpassungsmöglichkeiten an die jeweiligen individuellen Voraussetzungen der Kleinkinder möglich. Zudem kann mit der erfindungsgemäßen Spreizschiene dadurch, daß alle Teile aus einem für Röntgenstrahlen durchlässigen Material gefertigt sind, im angelegten Zustand eine Überprüfung der Stellung des Hüftgelenkes durch Röntgenaufnahmen ohne störende Reflexionen erfolgen. Der Aufbau ist außerdem leicht so ergänzbar, daß auch schwerste Fehlbildungen behandelt werden können.

### Liste der verwendeten Bezugszeichen

- 1,1': Spreizschiene
- 2: Beckenplatte
- 3: Beckengurthalter
- 4: Längsachse
- 5: Einstellbereich
- 6: Einstellbereich
- 7: Zentrum
- 8: Zentrum
- 9,9': Oberschenkelschalen
- 10,10': Oberschenkelschalen
- 11: Neigungswinkel
- 12,12: Neigungswinkel
- 13,13': Fußsockel
- 14,14': Fußsockel
- 15,15': Unterseite
- 16 16': Unterseite
- 17: Sockelbohrungen
- 18: Sockelbohrungen
- 19: Sockelbohrungen
- 20: Sockelbohrungen
- 21,21': Bohrungen
- 22,22': Bohrungen
- 23: Vorderseite
- 24: Stifte
- 25: Befestigungsschrauben
- 26: gedachte Linie
- 27: gedachte Linie
- 29: Rückseite
- 30: Senkungen
- 31: Senkungen
- 32: Polsterung
- 33: Polsterung
- 34: Führungsschlitze
- 35: Führungsschlitze
- 36: Gewinde
- 37: Gewinde
- 38,38': Halteschlitze
- 39,39': Halteschlitze
- 40: Innenseite
- 41: Senkkopf
- 42: Unterschenkelschale
- 43: Unterschenkelschale
- 44: Fläche
- 45: Führungsnut
- 46: Gegenstück
- 47: Gegenstück
- 48: Gegenfläche
- 49: Führungsnut
- 50: Gegenstück
- 51: Gegenstück
- 52: Schraube
- 53: Schraube
- 54: Schlitz
- 55: Schlitz
- 56: Schrauben
- 57: Schrauben

## Patentansprüche

1. Spreizschiene zur Korrektur von Fehlstellungen des Hüftgelenkes bei Kleinkindern, mit einer Beckenplatte mit einer Vorder- und einer Rückseite und einem daran angebrachten Beckengurthalter sowie mit zwei auf der Vorderseite angeordneten Oberschenkelschalen, dadurch gekennzeichnet, daß die gesamte Spreizschiene (1,1') aus einem von Röntgenstrahlen durchstrahlbaren Material hergestellt und die Beckenplatte (2) mit dem Beckengurthalter (3) einstückig ausgebildet und beidseitig einer Längsachse (4) um einen Einstellbereich (5,6) eines Beugungswinkels vergrößert ist, wobei die Beckenplatte (2) beidseitig ihrer Längsachse (4) in spiegelbildlicher Anordnung je ausgehend von einem Zentrum (7,8) und radial verlaufend eine Vielzahl von Bohrungen (21,22;21',22') aufweist, und daß mindestens die Oberschenkelschalen (9,9',10,10') je mit einem einen Neigungswinkel (11,12,12') aufweisenden Halter (13,14;13',14') verbunden sind und die Halter (13,14;13',14') je an einer ebenen Unterseite (15,16;15'16') an die Lage und den Durchmesser der Bohrungen (21,22;21',22') angepaßte Sockelbohrungen (17 bis 20) aufweisen, von denen mindestens eine mit einem Gewinde (37) versehen ist, wobei die Halter (13,14;13',14') auf der Vorderseite (23) der Beckenplatte (2) mindestens über eine Befestigungsschraube (25) befestigbar sind.

2. Spreizschiene nach Anspruch 1, dadurch gekennzeichnet, daß von den Zentren (7,8) je in entgegengesetzten Richtungen fächerförmig gedachte Linien (26,27) ausgehen, auf denen sich in radialer Verteilung die Bohrungen (21,22;21',22') befinden, wobei die gesamten Linien (26,27) oberhalb einer die Zentren (7,8) verbindenden Achse (28) einen Winkel von vorzugsweise 30° und unterhalb dieser Achse (28) einen Winkel von vorzugsweise 60° einnehmen.

3. Spreizschiene nach Anspruch 1 und 2, dadurch gekennzeichnet, daß die Zahl der Bohrungen (21,22;21',22') entlang jeder gedachten Linie (26,27) einer geraden Zahl entspricht und die Bohrungen (21,22;21',22') in zwei gleich große Gruppen geteilt sind, wovon die Bohrungen (21,22) mindestens einer Gruppe durchgehend sind und auf der Rückseite (29) der Beckenplatte (2) je eine Senkung (30,31) aufweisen, wobei die Sockelbohrungen (17 bis 20) der Halter (13,14) einen Abstand aufweisen, der mit den Bohrungen (21,22; 21'22') der Beckenplatte (2) korrespondiert.

4. Spreizschiene nach einem der Ansprüche 1 bis 3, dadurch gekennzeichnet, daß sie auf mindestens der Vorderseite (23) der Beckenplatte (12) mindestens teilweise mit einer hautfreundlichen, für Röntgenstrahlen durchlässigen Polsterung (32,33) versehen ist.

5. Spreizschiene nach einem der Ansprüche 1-4, dadurch gekennzeichnet, daß am Halter (13',14') eine Fläche (44) vorgesehen ist, die mit der Horizontalen einen Neigungswinkel (12') bildet, wobei diese Fläche (44) entlang ihrer steilsten Neigung mindestens eine erste Führungsnut (45) aufweist, in welche ein der Oberschenkelschale (9',10') zugeordnetes, entsprechend geformtes Gegenstück (46,47) eingesetzt ist, wobei die Oberschenkelschale (9',10') mit einer ebenen Gegenfläche (48) an der Fläche (44) anliegt und auf ihr verschiebbar und in gewünschter Position feststellbar ist.

6. Spreizschiene nach Anspruch 5, dadurch gekennzeichnet, daß parallel und im Abstand zur ersten Führungsnut (45) eine weitere Führungsnut (49) vorgesehen ist, in welche ein einer Unterschenkelschale (42,43) zugeordnetes, entsprechend geformtes Gegenstück (50,51) eingesetzt ist, wobei die Unterschenkelschale (42,43) in Richtung der Fläche verschiebbar und in gewünschter Position feststellbar ist.

7. Spreizschiene nach einem der Ansprüche 5 oder 6, dadurch gekennzeichnet, daß das Gegenstück (47,51) mit mindestens einer Schraube (52,53) an der zugeordneten Schale (9';42) befestigt ist.

8. Spreizschiene nach einem der Ansprüche 5 oder 6, dadurch gekennzeichnet, daß das Gegenstück (46;50) und die zugeordnete Schale (9';42) einstückig ausgebildet sind.

9. Spreizschiene nach einem der Ansprüche 1 bis 4, dadurch gekennzeichnet, daß mindestens die Oberschenkelschalen (9,10) und die Halter (13,14) einstückig ausgebildet sind.

10. Spreizschiene nach einem der Ansprüche 5 bis 8, dadurch gekennzeichnet, daß die Halter (13',14') in den Führungsnuten (45,49) geschlitzt sind, wobei die Schlitze (54,55) und damit die Führungsnuten (45,49) über quer eingesetzte Schrauben (56,57) zusammenziehbar sind zur Festklammerung der jeweils geführten Schale.
